# EUROPÄISCHE PATENTSCHRIFT

(11) **EP 3 380 018 B1**
(45) Veröffentlichungstag und Bekanntmachung des Hinweises auf die Patenterteilung: **11.10.2023**
(21) Anmeldenummer: 16816208.9
(22) Anmeldetag: 24.11.2016
(51) Int. Cl.: A61B 17/12

(54) **BANDFÖRMIGES OKKLUSIONSMITTEL**
BAND-SHAPED OCCLUSION MEANS
MOYEN D'OCCLUSION EN FORME DE BANDE

(30) Priorität: 26.11.2015 DE 102015120554
(43) Veröffentlichungstag der Anmeldung: 03.10.2018
(73) Patentinhaber: femtos GmbH, 44799 Bochum (DE)
(72) Erfinder: MONSTADT, Hermann, 44797 Bochum (DE); HENKES, Hans, 70192 Stuttgart (DE); HANNES, Ralf, 44137 Dortmund (DE)
(74) Vertreter: Schneiders & Behrendt Bochum
(86) Internationale Anmeldenummer: PCT/EP2016/078658
(87) Internationale Veröffentlichungsnummer: WO 2017/089451

(56) Entgegenhaltungen:
- CN-A- 104 257 412
- US-A1- 2006 241 680
- US-A1- 2012 245 614
- US-A1- 2013 184 658

## Beschreibung

Die Erfindung betrifft ein Implantat für den vaskulären Einsatz, das durch einen Katheter transportiert werden kann und insbesondere dazu bestimmt ist, den Blutfluss im Bereich arteriovenöser Fehlbildungen, wie etwa Fisteln und Aneurysmen, zu beeinflussen, die Aussackung der Fehlbildung zu füllen und die Thrombenbildung zu begünstigen.

Aneurysmen sind zumeist sackartig ausgebildete Erweiterungen der Gefäßwand, die vornehmlich an strukturell geschwächten Stellen der Gefäßwand durch den konstanten Druck des Blutes entstehen. Entsprechend empfindlich sind die Gefäßinnenwände eines Aneurysmas und anfällig für Verletzungen, die bis zur Ruptur des Aneurysmasackes führen können. In der Folge kann es zu erheblichen gesundheitlichen Beeinträchtigungen, wie neurologischen Ausfällen, bis hin zum Tod des Patienten kommen. Dies gilt vor allem für derartige Fehlbildungen im zerebralen Bereich.

Aktuelle intravaskuläre Methoden zur Behandlung von Aneurysmen sind hinreichend bekannt und verfolgen vor allem zwei Ansätze. Entweder wird das Aneurysma mit einem Okklusionsmittel ausgefüllt, hier sind aus dem Stand der Technik verschiedene Füllstoffe und Techniken bekannt, oder man verschließt den Zugang zum Aneurysma, etwa den Hals eines Beerenaneurysmas, von der Blutgefäßseite aus durch Stent-ähnliche Implantate. Beide Verfahren dienen dazu, den Blutfluss in das Aneurysma und somit den Druck auf das Aneurysma zu vermindern, im Idealfall zu eliminieren.

Das Füllmaterial, in der Regel handelt es sich um Platinspiralen (coils), füllt den Aneurysmasack aus und thrombogenisiert in der Folge zu einem mehr oder weniger kompakten Gebilde innerhalb des Aneurysmas. Zwei Probleme dieser Behandlungsmethode sind bekannt und können im weiteren Krankheitsverlauf zu Nachteilen führen. Einerseits füllt man das Aneurysma nur unzureichend aus, was die Blutzufuhr in das Aneurysma und somit einen weiterhin bestehenden Druck auf dessen Innenwand zulässt. Die Gefahr einer stetigen Erweiterung des Aneurysmas und schließlich seiner Ruptur besteht, wenn auch in abgeschwächter Form, weiterhin. Andererseits ist die Behandlungsmethode nur für Aneurysmen mit relativ engem Hals - sogenannten Beerenaneurysmen - geeignet, da ansonsten die Gefahr besteht, dass die *coils* aus einem weiten Aneurysmahals in das Blutgefäß ragen und dort thrombogenisieren, was zu Verschlüssen führen kann. Schlimmstenfalls wird ein *coil* vollständig aus dem Aneurysma geschwemmt und verschließt Gefäße an anderer Stelle.

Um die *coils* an ihrem Platz im Aneurysmasack zu halten, wird der Aneurysmahals häufig zusätzlich mit einem speziellen Stent abgedeckt.

Ein anderer intravaskulärer Behandlungsansatz setzt auf sogenannte Flussumlenker (*flow diverter*). Diese Implantate ähneln in ihrer äußeren Erscheinung Stents, die zur Behandlung von Stenosen eingesetzt werden. Da die Aufgabe der *flow diverter* jedoch nicht das Offenhalten eines Gefäßes, sondern der Verschluss des Aneurysmazugangs auf Seiten des Blutgefäßes ist, ist die Maschenweite sehr eng, alternativ sind diese Implantate mit einer Membran überzogen. Nachteilig bei diesen Implantaten ist die Gefahr, dass abgehende Seitenäste in unmittelbarer Nähe des zu behandelnden Aneurysmas mitunter ebenfalls abgedeckt werden und dadurch mittel- oder langfristig verschlossen werden.

In der WO 2012/034135 A1 ist ein Implantat offenbart, das - in verschiedenen Ausführungsformen - bei seiner Freisetzung innerhalb des Aneurysmas eine dreidimensionale, annähernd sphärische Form annimmt und so das Aneurysma ausfüllt. Ausgangsmaterial für das dreidimensionale Implantat ist in jedem Fall ein maschenartiges Gewebe, die Ausführungsbeispiele und Abbildungen sind allesamt auf ein röhrenartiges Geflecht aus Formgedächtnismaterial bezogen.

Für den expandierten, dreidimensionalen Zustand des Implantates bedeutet dies, dass teilweise 4 und mehr Gewebsschichten übereinander liegen, was dem nahezu runden Implantat eine unvorteilhafte Steifigkeit verleiht. Aneurysmen sind selten absolut rund, ein dreidimensionales Implantat sollte aber in der Lage sein, sich in die Morphologie des Aneurysmas bestmöglich einzupassen. Zudem erweist es sich für Katheter eines geringen Kalibers als zu voluminös.

US 2006/241680 A1 offenbart eine beschichtete endoluminale Filtervorrichtung mit einer Stützstruktur und einer daran angebrachten Filterstruktur.

CN 104 257 412 A offenbart eine Vorrichtung aus einzelnen Elementen zur Behandlung zerebraler Aneurysmen, wobei zwischen den Drähten der Vorrichtung eine durch Elektrospinnen erhaltene Nano-Membran gespannt sein kann, die eine Thrombenbildung und hierdurch den Verschluss eines Aneurysmas fördert.

US 2012/245614 A1 offenbart einen Okkluder, der ein oder mehrere durch zumindest einen Draht gebildete scheibenförmige Elemente umfasst, die mit einer Membran bespannt sind. Der Okkluder dient zum Verschluss von Gefäßen.

US 2013/184658 A1 offenbart ebenfalls einen Okkluder aus einem Draht mit Formgedächtniseigenschaften und daran befestigtem membranösen Material.

Der Erfindung liegt somit die Aufgabe zu Grunde, ein Okklusionsmittel für die intrasakkuläre Behandlung eines vaskulären Aneurysmas zur Verfügung zu stellen, das nach der Expansion eine definierte, jedoch gleichzeitig möglichst flexible dreidimensionale Gestalt annimmt. Zusätzlich soll das Implantat die Thrombusbildung innerhalb des Aneurysmas begünstigen und weitere Behandlungsoptionen offen halten.

Diese Aufgabe wird erfindungsgemäß durch ein Implantat der eingangs genannten Art gelöst, bei dem die Okklusionseinheit nach der Expansion eine dreidimensionale Gestalt annimmt und anschließend mittels bekannter mechanischer, elektrolytischer oder thermischer Trennung beziehungsweise Auflösung der Verbindungsstelle von der Einführeinheit abgetrennt wird. Die Okklusionseinheit besteht aus einem Gerüst, das aus einer Mehrzahl von Streben geformt wird, zwischen denen sich eine Bespannung befindet. Das Gerüst kann aus einer Vielzahl von Drähten geformt oder durch Laserschneiden gewonnen werden. In jedem Fall sollte das Material Formgedächtniseigenschaften besitzen, denkbar ist hier insbesondere Nitinol. Bei der Bespannung handelt es sich vorzugsweise um eine Polymer-Membran, insbesondere eine durch Elektrospinnen gewonnene Nanomembran, es ist aber auch jede andere geeignete Membran oder Bespannung, beispielsweise aus einer Vielzahl von geflochtenen oder ungeflochtenen Drähten, denkbar. Die Drähte können ihrerseits mit einer Membran abgedeckt sein.

Die erfindungsgemäße Okklusionseinheit weist in der gestreckten Anordnung eine zentrale Axialstrebe (*spine*) und eine oder eine Vielzahl von daran befestigten Bogenstreben auf. Axialstrebe und/oder Bogenstrebe/n bestehen aus einem Formgedächtnismaterial wie beispielsweise Nitinol. Sie können auch aus anderen Materialien, wie beispielsweise Polymeren, gefertigt sein.

Der Axialstrebe kann eine sphärische oder helikale Form aufgeprägt sein, jedoch ist auch jede andere Form denkbar. Bevorzugt sind all jene Formen, die zur gewünschten dreidimensionalen Ausformung des Implantates führen und sich bei der intrasakkulären Expansion als vorteilhaft erweisen. Hierzu gehören insbesondere auch Formen, bei denen sich das proximale und distale Ende der Okklusionseinheit nach Einbringen in den Aneurysmasack in das Lumen der expandierten Okklusionseinheit formen, um so eine Verletzung der Aneurysmawand zu verhindern.

Das erfindungsgemäße Implantat umfasst im gestreckten Zustand einen zusätzlichen, nach der Implantation zurückziehbaren Stützdraht, der die Axialstrebe für die Passage durch den Katheter in ihrer Form unterstützt. Diese Anordnung kann den Transport des gestreckten Implantates durch einen Katheter vereinfachen. Der Stützdraht verläuft während des Transportes parallel zum Implantat und besitzt keine aufgeprägte dreidimensionale Form.

In einer Variante der ersten Ausführungsform kann die Axialstrebe in ihrem gestreckten Zustand auch eine Spirale sein, die sich mindestens auf die Länge einer Bogenstrebe dehnen lässt. Der Stützdraht kann in diesem Fall zentral durch die Seele der Spirale verlaufen und die Spirale auf der gewünschten Dehnung fixieren. Nach Expansion ist der Stützdraht zurückziehbar und ermöglicht es der Axialstrebe, ihre dreidimensionale Gedächtnisform einzunehmen, und verbleibt nicht im Implantat. Die Formeigenschaften der Spirale entsprechen denen der zuvor beschriebenen soliden Variante.

In einer weiteren Variante der ersten Ausführungsform kann die Axialstrebe eine Röhre sein. Der Stützdraht kann in diesem Fall zentral durch die Röhre verlaufen und die Röhre in ihrem gestreckten Zustand halten. Nach Expansion ist der Stützdraht zurückziehbar und ermöglicht es der röhrenförmigen Axialstrebe so, ihre dreidimensionale Gedächtnisform einzunehmen, und verbleibt nicht im Implantat. Die Formeigenschaften der Röhre entsprechen denen der beschriebenen soliden Variante.

Die Bogenstrebe oder Bogenstreben können im (teil-)expandierten Zustand sinusoidal verlaufen. Stellt man sich Bogenstrebe in einem Koordinatenkreuz vor, so bildet die Axialstrebe die x-Achse und die Bogenstrebe die Sinuskurve. An den Schnittpunkten von Axialstrebe und Bogenstrebe kann diese an der Axialstrebe fixiert sein. Die Form, die von der Axialstrebe und einer darüber liegenden Kurve der Bogenstrebe gebildet wird, wird im Folgenden als Flügel (*wings*) bezeichnet. Bezieht man sich - lediglich aus Veranschaulichungszwecken - auf eine Sinuswelle, so entsprechen die Kurve der Bogenstrebe einer halben Sinusschwingung und der dazugehörige Abschnitt der Axialstrebe einer halben Periodenlänge. Zur Vereinfachung soll im Folgenden bei der Fläche eines Flügels nur noch von Axialstrebenabschnitt und Bogenstrebenkurve gesprochen werden. Ebenso soll nachfolgend die Größe eines Flügels über das Verhältnis seiner Breite (des Axialstrebenabschnitts) zu seiner Länge (der maximalen Amplitude des dazugehörigen Abschnitts der Bogenstrebenkurve) definiert werden.

Besteht die Okklusionseinheit aus einer Axialstrebe und nur einer Bogenstrebe, so werden 2 bis 30, bevorzugt 4 bis 10 dieser Flügel gebildet. Die Anzahl der Flügel ist jedoch auch abhängig von der Größe des Implantates. Es kann sinnvoll sein, Implantate mit größeren Durchmessern für große Aneurysmen auch mit mehr als 30 Flügeln herzustellen.

In einer anderen Variante der ersten Ausführungsform können auch zwei Bogenstreben so an einer Axialstrebe befestigt sein, dass die Flügel wechselständig angeordnet sind. Die zweite Bogenstrebe verläuft dann insbesondere achsensymmetrisch zur Axialstrebe. Die von beiden Bogenstreben gebildeten Flügel liegen bevorzugt, jedoch nicht zwingend in einer Ebene. Besteht die Okklusionseinheit aus einer Axialstrebe und zwei Bogenstreben, so werden 4 bis 40, bevorzugt 8 bis 20 dieser Flügel gebildet. Denkbar sind Ausführungsformen mit 40 oder mehr Flügeln bei Implantaten mit einem großen Durchmesser für große Aneurysmen.

Ausführungen mit einer Vielzahl von Bogenstreben und einer entsprechenden Vielzahl von Flügeln sind ebenso denkbar. Auch ist es denkbar, dass die Flügel nicht in nur einer Ebene liegen, sondern beispielsweise spiralig oder in einer anderen Weise um die Axialstrebe angeordnet sind, um die gewünschte dreidimensionale Form im expandierten Zustand zu realisieren.

Die Flügel können von gleicher oder unterschiedlicher Größe sein. Sind sie sinusoidal ausgestaltet, können sich die Amplitude und/oder Periodenlänge von proximal nach distal - oder auch umgekehrt - verringern. Auch ist eine alternierende Größenänderung oder eine einem anderen Muster folgende Anordnung denkbar.

Neben der sinusoidalen Form sind auch alle anderen Formen denkbar.

Die über das Verhältnis des Axialstrebenabschnitts zur maximalen Amplitude des dazugehörigen Abschnitts der Bogenstrebenkurve definierte Größe eines Flügels variiert je nach Ausführungsform.

Die Länge eines Flügels wird in der Regel von ¼(2πr) nicht wesentlich abweichen, wobei hier r=d/2 und d der Durchmesser des Implantats sind. Einzelne Flügel desselben Implantates können von dieser Länge gegebenenfalls auch stärker abweichen, wenn dies zur Ausbildung der gewünschten dreidimensionalen Form vorteilhaft ist.

Die Breite des entsprechenden Axialstrebenabschnitts hängt von der Anzahl der in dem Implantat zu realisierenden Flügel ab. Bei angenommener gleicher Breite aller Flügel bedeutet dies für n Flügel eine Breite von (2πr)/2n bei wechselständiger Anordnung beziehungsweise (2πr)/n bei gegenständiger Anordnung. Sofern sich einzelne Flügel überlappen sollen, kann sich die Breite weiter reduzieren. Einzelne Abschnitte desselben Implantates können von dieser Breite gegebenenfalls auch stärker abweichen, wenn dies zur Ausbildung der dreidimensionalen Form von Vorteil ist.

Die Fläche der Flügel wird durch eine Bespannung bedeckt. Hierbei handelt es sich um eine Polymer-Membran, bevorzugt um einen Polyurethanfilm oder um eine Nanomembran aus Polycarbonaturethan (PCU), die durch Elektrospinnen erzeugt wird. Die Membran kann an Bogenstrebe und Axialdraht oder auch nur an der Bogenstrebe fixiert sein. Ist der Axialdraht als Spirale ausgeführt, bietet es sich an, die Membran nur an der Bogenstrebe zu befestigen. Es ist aber auch eine Bespannung mit einem Polymergeflecht, Polymerfäden, Drähten oder einem Drahtgitter oder Drahtgeflecht denkbar und möglich.

Um die Thrombogenität des Implantats zu erhöhen, können in die Membran thrombogene Materialien, wie beispielsweise Nylonfilamente, eingesponnen oder eingeflochten werden. Die Membran, die Bespannung oder gegebenenfalls die Bogenstreben oder der Axialdraht können mit thrombogenen Substanzen beschichtet werden.

Die Nylonfilamente können zur Stabilisierung der Membran mit je einem Ende an Bogenstrebe und/oder Axialstrebe befestigt werden. In einer bevorzugten Ausführungsform verlaufen die Filamente senkrecht zur Axialstrebe. Jeder andere Verlauf ist jedoch auch denkbar. In einer weiteren bevorzugten Ausführungsform verlaufen die Filamente parallel zur Axialstrebe und sind mit ihren Enden jeweils nur an der Bogenstrebe fixiert. Es sind auch Ausführungsformen denkbar, die beide Möglichkeiten kombinieren.

Ist die Axialstrebe als Spirale ausgeführt, bietet es sich an, die Filamente nur an der Bogenstrebe zu befestigen.

Die expandierte Form der Okklusionseinheit ist dreidimensional. Hierbei sind kugelförmige, sphärische oder auch ovoide Figuren denkbar. Auch amorphe Formen sind denkbar, ebenso helikale Strukturen. Die Flügel der Okklusionseinheit können sich, müssen sich in der expandierten dreidimensionalen Form aber nicht überlappen.

Um die Okklusionseinheit für den Transport durch den Katheter in eine gestreckte Form zu bringen, müssen die Flügel an die Axialstrebe gelegt werden. Hierzu sind verschiedene Möglichkeiten denkbar. Die Axialstrebe kann, wie bereits beschrieben, eine Spirale sein, die sich auf die Länge der Bogenstreben dehnen lässt. Ebenso wäre es denkbar, dass die Bogenstreben beweglich an der Axialstrebe befestigt sind oder dass die Flügel um die Axialstrebe aufgerollt werden.

Die Erfindung wird anhand der folgenden Zeichnung näher erläutert. Es zeigt:

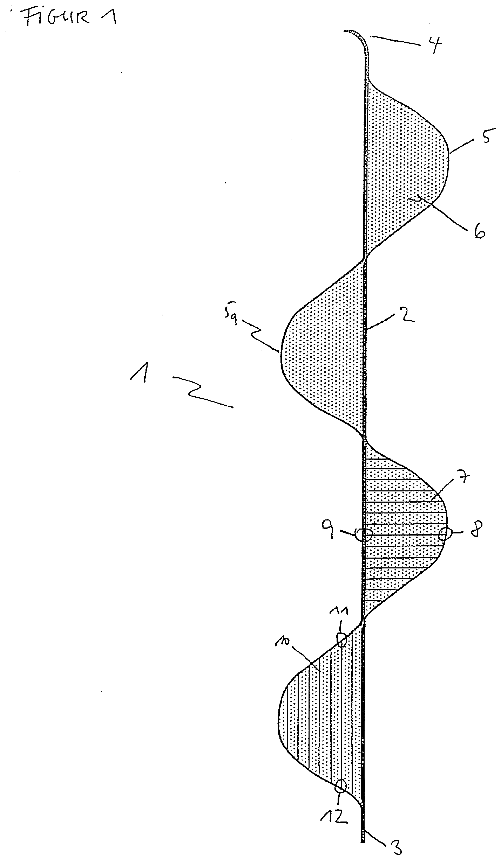

- Figur 1: eine erste Ausführungsform der Okklusionseinheit 1 des erfindungsgemäßen Implantats in teilexpandierter Ansicht außerhalb eines Katheters bestehend aus einer Axialstrebe 2, einer Bogenstrebe 5 mit Bespannung 6 und Nylonfilamenten 7, 10.

Figur 1 zeigt die erste Ausführungsform 1 der Okklusionseinheit des erfindungsgemäßen Implantats in teilexpandierter, gestreckter, zweidimensionaler Ansicht außerhalb des Katheters, ohne dass die endgültige dreidimensionale Form angenommen wurde. Die zentral verlaufende Axialstrebe 2 mit proximalem 3 und distalem 4 Ende, einer die Flügel 5a bildenden Bogenstrebe 5 mit Membran 6 und Nylonfilamenten 7, 10, die - bei zur Axialstrebe 2 senkrechter Anordnung 7 - an der Bogenstrebe 5, 8 und an der Axialstrebe 2, 9 befestigt sind, oder bei zur Axialstrebe 2 paralleler 10 Anordnung mit beiden Enden 11, 12 an der Bogenstrebe 5 befestigt sind.

## Patentansprüche

1. Implantat zur Behandlung arteriovenöser Fehlbildungen, insbesondere Aneurysmen, bestehend aus einer Einführeinheit und einer davon trennbaren Okklusionseinheit (1), wobei die Okklusionseinheit (1) aus mehreren Untereinheiten (5a) besteht, wobei die Untereinheiten (5a) aus einem Gerüst mit Formgedächtniseigenschaften bestehen, das aus Draht gebildet oder durch Laserschneiden hergestellt wird, wobei das Gerüst aus Streben (5) aufgebaut ist und die Okklusionseinheit (1) eine erste gestreckte Anordnung zum Transport durch einen Katheter und eine zweite expandierte Anordnung nach Austritt aus dem Katheter aufweist, wobei das Implantat in der expandierten Anordnung der Okklusionseinheit (1) eine dreidimensionale Gestalt annimmt, wobei sich zwischen den Streben (5) des Gerüstes der Okklusionseinheit (1) eine Bespannung (6) befindet, **dadurch**
**gekennzeichnet, dass** das Gerüst der Okklusionseinheit (1) aus einer zentralen Axialstrebe (2) und einer oder mehreren daran befestigten Bogenstreben (5) besteht und einen zusätzlichen Stützdraht umfasst, wobei die Axialstrebe (2) für die Passage durch den Katheter und zur anschließenden Freisetzung im Aneurysma im gestreckten Zustand durch den zusätzlichen Stützdraht unterstützt wird und der zusätzliche Stützdraht nach Freisetzung zurückziehbar ist.

2. Implantat nach Anspruch 1, **dadurch gekennzeichnet, dass** es sich bei der Bespannung (6) um eine Membran, insbesondere eine aus Polymer hergestellte Membran, handelt.

3. Implantat nach Anspruch 2, **dadurch gekennzeichnet, dass** es sich bei der Membran (6) um eine durch Elektrospinnen erzeugte Nanomembran handelt.

4. Implantat nach Anspruch 1, **dadurch gekennzeichnet, dass** es sich bei der Bespannung (6) um eine Bespannung aus Drähten handelt, wobei die Drähte geflochten oder nicht geflochten sein können.

5. Implantat nach einem der vorherigen Ansprüche, **dadurch gekennzeichnet, dass** die Bogenstreben (5) im expandierten Zustand gegen- oder wechselständig angeordnete Flügel (5a) in sinusoidaler oder in sonstiger Form ausbilden, wobei die Axialstrebe (2) der x-Achse entspricht und die Bogenstrebe (5) einer Funktionskurve entspricht und die Fläche eines Flügels (5a) der Fläche entspricht, die zwischen zwei Schnittpunkten der Funktionskurve mit der x-Achse liegt und von den entsprechenden Abschnitten der Funktionskurve und der x-Achse eingeschlossen wird.

6. Implantat nach Anspruch 5, **dadurch gekennzeichnet, dass** sich an der Axialstrebe (2) bei wechselseitiger Anordnung wenigstens 3 Flügel (5a) befinden und bei gegenständiger Anordnung wenigstens 6 Flügel (5a) befinden.

7. Implantat nach Anspruch 6, **dadurch gekennzeichnet, dass** die Flügel (5a) im expandierten Zustand eine identische Form und/oder Größe aufweisen.

8. Implantat nach Anspruch 6, **dadurch gekennzeichnet, dass** die Flügel (5a) im expandierten Zustand eine unterschiedliche Form und/oder Größe aufweisen, insbesondere dass sich bei sinusoidaler Form die Amplitude und/oder die Periodenlänge von proximal nach distal oder von distal nach proximal verringert.

9. Implantat nach Anspruch 6, **dadurch gekennzeichnet, dass** die Flügel (5a) im expandierten Zustand eine unterschiedliche Form und/oder Größe aufweisen, wobei Form und/oder Größe periodisch alternieren oder sich nach einem anderen Muster ändern.

10. Implantat nach einem der vorherigen Ansprüche, **dadurch gekennzeichnet, dass** die durch die Bogenstrebe (5) im expandierten Zustand aufgespannte Fläche (5a) zusätzlich durch ein oder mehrere nur an der Bogenstrebe befestigte Filamente (10) verstärkt ist, die vorzugsweise parallel (10) zur Axialstrebe verlaufen.

11. Implantat nach einem der vorherigen Ansprüche, **dadurch gekennzeichnet, dass** die durch die Bogenstrebe (5) im expandierten Zustand aufgespannte Fläche (5a) durch ein oder mehrere Filamente (7) verstärkt ist, wobei ein Ende des jeweiligen Filaments mit der Bogenstrebe (5) und das andere mit der Axialstrebe (2) verbunden ist, sodass das Filament vorzugsweise senkrecht (7) zur Axialstrebe ausgerichtet ist.

12. Implantat nach einem der Ansprüche 1-10, **dadurch gekennzeichnet, dass** die Axialstrebe (2) eine dehnbare Spirale ist, wobei die gedehnte Länge im gestreckten Zustand für den Transport durch einen Katheter annähernd der Länge einer Bogenstrebe (5) im gestreckten Zustand entspricht.

13. Implantat nach einem der vorherigen Ansprüche, **dadurch gekennzeichnet, dass** die Okklusionseinheit (1) im expandierten Zustand eine kugelförmige oder ovoide Form ausbildet.

14. Implantat nach einem der vorherigen Ansprüche, **dadurch gekennzeichnet, dass** die Membran (6) thrombogene Zusätze enthält.

15. Implantat nach Anspruch 14, **dadurch gekennzeichnet, dass** die thrombogenen Zusätze Nylonfilamente sind.

## Claims

1. Implant for the treatment of arteriovenous malformations, in particular aneurysms, consisting of an insertion unit and an occlusion unit (1 ) separable therefrom, wherein the occlusion unit (1) consists of several subunits (5a), wherein the subunits (5a) consist of a framework with shape memory properties, which is formed from wire or produced by laser cutting, wherein the framework is composed of struts (5) and the occlusion unit (1) has a first elongated arrangement for transport through a catheter and a second expanded arrangement after exit from the catheter, wherein the implant takes on a three-dimensional shape in the expanded arrangement of the occlusion unit (1), wherein a covering (6) is located between the struts (5) of the framework of the occlusion unit (1), **characterized in that** the framework of the occlusion unit (1) consists of a central axial strut (2) and one or more arc struts (5) attached thereto and comprises an additional support wire, wherein the axial strut (2) is supported by the additional support wire for passage through the catheter and for subsequent release in the aneurysm in the elongated state and the additional support wire is retractable after release.

2. Implant according to claim 1, **characterized in that** the covering (6) is a membrane, in particular a membrane made of polymer.

3. Implant according to claim 2, **characterized in that** the membrane (6) is a nanomembrane produced by electrospinning.

4. Implant according to claim 1, **characterized in that** the covering (6) is a covering of wires, wherein the wires may be braided or non-braided.

5. Implant according to any one of the preceding claims, **characterized in that** the arc struts (5) in the expanded state form oppositely or alternately arranged wings (5a) in sinusoidal or other form, wherein the axial strut (2) corresponds to the x-axis and the arc strut (5) corresponds to a functional curve and the area of a wing (5a) corresponds to the area which lies between two points of intersection of the functional curve with the x-axis and is enclosed by the corresponding sections of the functional curve and the x-axis.

6. Implant according to claim 5, **characterized in that** at least 3 wings (5a) are located on the axial strut (2) in the case of an alternating arrangement and at least 6 wings (5a) are located in the case of an opposed arrangement.

7. Implant according to claim 6, **characterized in that** the wings (5a) have an identical shape and/or size in the expanded state.

8. Implant according to claim 6, **characterized in that** the wings (5a) in the expanded state have a different shape and/or size, in particular that the amplitude and/or the period length decreases from proximal to distal or from distal to proximal in the case of a sinusoidal shape.

9. Implant according to claim 6, **characterized in that** the wings (5a) in the expanded state have a different shape and/or size, wherein the shape and/or size alternate periodically or change according to a different pattern.

10. Implant according to any one of the preceding claims, **characterized in that** the area (5a) spanned by the arc strut (5) in the expanded state is additionally reinforced by one or more filaments attached only to the arc strut, which filaments preferably run parallel (10) to the axial strut.

11. Implant according to any one of the preceding claims, **characterized in that** the area (5a) spanned by the arc strut (5) in the expanded state is reinforced by one or more filaments (7), one end of the respective filament being connected to the arc strut (5) and the other to the axial strut (2), so that the filament is preferably aligned perpendicularly (7) to the axial strut.

12. Implant according to any of the claims 1-10, **characterized in that** the axial strut (2) is a stretched spiral, wherein the stretched length in the elongated state for transport through a catheter approximately corresponds to the length of an arc strut (5) in the elongated state.

13. Implant according to any one of the preceding claims, **characterized in that** the occlusion unit (1) forms a spherical or ovoid shape in the expanded state.

14. Implant according to any one of the preceding claims, **characterized in that** the membrane (6) contains thrombogenic additives.

15. Implant according to claim 14, **characterized in that** the thrombogenic additives are nylon filaments.

## Revendications

1. Implant pour le traitement de malformations artério-veineuses, en particulier d'anévrismes, constitué d'une unité d'insertion et d'une unité d'occlusion (1) séparable de celle-ci, l'unité d'occlusion (1) étant constituée de plusieurs sous-unités (5a), les sous-unités (5a) étant constituées d'une ossature ayant des propriétés de mémoire de forme, qui est formée de fil ou fabriquée par découpe au laser, l'ossature étant constituée de entretoises (5) et l'unité d'occlusion (1) présentant un premier agencement étiré pour le transport par un cathéter et un deuxième agencement expansé après la sortie du cathéter, l'implant prenant une forme tridimensionnelle dans l'agencement expansé de l'unité d'occlusion (1), un revêtement (6) étant situé entre les entretoises (5) de l'ossature de l'unité d'occlusion (1),
**caractérisé en ce que**
l'ossature de l'unité d'occlusion (1) est constituée d'une entretoise axiale centrale (2) et d'une ou plusieurs entretoises arquées (5) fixées à celle-ci et comprend un fil de soutien supplémentaire, l'entretoise axiale (2) étant soutenue par le fil de soutien supplémentaire pour le passage à travers le cathéter et pour la libération ultérieure dans l'anévrisme à l'état étiré, et le fil de soutien supplémentaire pouvant être retiré après la libération.

2. Implant selon la revendication 1, **caractérisé en ce que** le revêtement (6) est une membrane, en particulier une membrane fabriquée en polymère.

3. Implant selon la revendication 2, **caractérisé en ce que** la membrane (6) est une membrane de type nano membrane obtenue par électrofilage.

4. Implant selon la revendication 1, **caractérisé en ce que** le revêtement (6) est un revêtement de fils métalliques, les fils pouvant être tressés ou non.

5. Implant selon l'une des revendications précédentes, **caractérisé en ce que** les entretoises arquées (5) forment, à l'état expansé, des ailes (5a) disposées en opposition ou en alternance, de forme sinusoïdale ou autre, l'entretoise axiale (2) correspondant à l'axe x et l'entretoise arquée (5) correspondant à une courbe fonctionnelle et la surface d'une aile (5a) correspondant à la surface qui se trouve entre deux points d'intersection de la courbe fonctionnelle avec l'axe x et qui est incluse dans les sections correspondantes de la courbe fonctionnelle et de l'axe X.

6. Implant selon la revendication 5, **caractérisé en ce que** à l'entretoise axiale (2) se trouve au moins 3 ailes (5a) dans le cas d'une disposition alternée et au moins 6 ailes (5a) dans le cas d'une disposition oppose.

7. Implant selon la revendication 6, **caractérisé en ce que** les ailes (5a) présentent une forme et/ou une taille identique à l'état expansé.

8. Implant selon la revendication 6, **caractérisé en ce que** les ailes (5a) présentent une forme et/ou une taille différente à l'état expansé, en particulier **en ce que**, dans le cas d'une forme sinuso dale, l'amplitude et/ou la longueur de la période diminue de proximal à distal ou de distal à proximal.

9. Implant selon la revendication 6, **caractérisé en ce que** les ailes (5a) présentent une forme et/ou une taille différente à l'état expansé, la forme et/ou la taille alternant périodiquement ou changeant selon un autre modèle.

10. Implant selon l'une des revendications précédentes, **caractérisé en ce que** la surface (5a) tendue par l'entretoise courbe (5) à l'état expansé est en outre renforcée par un ou plusieurs filaments fixés uniquement à l'entretoise courbe, qui s'étendent de préférence parallèlement (10) à l'entretoise axiale.

11. Implant selon l'une des revendications précédentes, **caractérisé en ce que** la surface (5a) tendue par l'entretoise arquée (5) à l'état expansé est renforcée par un ou plusieurs filaments (7), une extrémité de chaque filament étant reliée à l'entretoise arquée (5) et l'autre à l'entretoise axiale (2), de sorte que le filament est de préférence orienté perpendiculairement (7) à l'entretoise axiale.

12. Implant selon l'une quelconque des revendications 1 à 10, **caractérisé en ce que** l'entretoise axiale (2) est une spirale extensible, la longueur étirée à l'état étiré pour le transport par un cathéter correspondant approximativement à la longueur d'une entretoise courbe (5) à l'état étiré.

13. Implant selon l'une des revendications précédentes, **caractérisé en ce que** l'unité d'occlusion (1) présente une forme sphérique ou ovoide à l'état expanse.

14. Implant selon l'une des revendications précédentes, **caractérisé en ce que** la membrane (6) contient des additifs thrombogènes.

15. Implant selon la revendication 14, **caractérisé en ce que** les additifs thrombogènes sont des filaments de nylon
